# EUROPÄISCHE PATENTSCHRIFT

(11) **EP 0 416 236 B1**
(45) Veröffentlichungstag und Bekanntmachung des Hinweises auf die Patenterteilung: **08.11.1995**
(21) Anmeldenummer: 90112744.9
(22) Anmeldetag: 04.07.1990
(51) Int. Cl.: C01C 3/00, C01C 1/12, C07C 273/18, C07C 269/02, C07C 333/04, C07C 265/08

(54) **Verfahren zur Abtrennung von Isocyansäure aus einem Gemisch von Isocyansäure und Ammoniak**
Process for the isolation isocyanic acid from a gaseous mixture of isocyanic acid and ammonia
Procédé pour l'isolation d'acide cyanique d'un mélange gazeux d'acide cyanique et d'ammoniac

(30) Priorität: 28.07.1989 AT 1828/89; 28.07.1989 AT 1829/89
(43) Veröffentlichungstag der Anmeldung: 13.03.1991
(73) Patentinhaber: Chemie Linz GmbH, A-4021 Linz (AT)
(72) Erfinder: Müllner, Martin, Dipl.-Ing. Dr., A-4050 Traun (AT); Stern, Gerhard, Dipl.-Ing. Dr., A-4180 Sonnberg (AT); Erich, Schulz, A-4052 Ansfelden (AT)
(74) Vertreter: Kunz, Ekkehard, Dr.

(56) Entgegenhaltungen:
- EP-A- 0 124 704
- US-A- 3 950 497
- CHEMICAL ABSTRACTS, Band 82, Nr. 26, 30 Juni 1975, Columbus, OH (US); I. TAKAHASHI et al., Seite 129, Nr. 173135u

## Beschreibung

Die Erfindung betrifft ein Verfahren zur Abtrennung von Isocyansäure aus einem gasförmigen Gemisch aus Isocyansäure und Ammoniak in Form eines Adduktes aus Isocyansäure und tertiärem Amin oder aus Isocyansäure und Ether.

Gemische aus Isocyansäure und Ammoniak dienen u. a. als Ausgangsmaterial für die Synthese von Melamin und werden durch thermische Zersetzung von Harnstoff, beispielsweise nach EP-A-0 124 704 erhalten.
Isocyansäure selbst wäre ein wertvoller, reaktiver C-1 Baustein für die Synthese einer Vielzahl organischer Verbindungen . Ihre Verwendung für diesen Zweck ist jedoch beschränkt, da sie nicht sehr stabil ist und leicht zu linearen und cyclischen Polymeren polymerisiert. Außerdem ist sie nur auf aufwendigem Weg herstellbar. So ist in Chemical Abstracts, Vol.81 (1974) 172444m und Vol. 82 (1975), 173135u die Bildung von Isocyansäure durch thermische Zersetzung von Harnstoff unter Abtrennung von Ammoniak beschrieben, wobei jedoch ein Feststoff, nämlich Cyanursäure hergestellt wird, der bei 330 bis 600 °C zu Isocyansäure zersetzt werden muß. Dabei ist bekannt, daß die Zersetzung von Cyanursäure nur langsam und unvollständig verläuft, da Cyanursäure im Vergleich zu Isocyansäure sehr stabil ist.

Es wurde nun gefunden, daß bei der Abtrennung von Isocyansäure aus einem gasförmigen Gemisch aus Isocyansäure und Ammoniak der Umweg über die Cyanursäure vermieden werden kann, wenn man dem Gemisch aus Isocyansäure und Ammoniak ein tertiäres Amin oder einen Ether zusetzt, das entstandene, gasförmige Reaktionsgemisch abkühlt und kondensiert. Dabei bildet sich unerwarteterweise trotz der Anwesenheit des Ammoniaks in der Reaktionsmischung nicht Ammoniumisocyanat, sondern es entsteht ein Addukt der Isocyansäure mit dem jeweils verwendeten tertiären Amin oder dem jeweils verwendeten Ether. Das Ammoniak bleibt gasförmig und kann abgetrennt werden. Dabei sind die Addukte aus Isocyansäure und tertiärem Amin und die Addukte aus Isocyansäure und Ether stabiler als freie Isocyansäure. Die Addukte können zur Weiterreaktion mit verschiedenen Substraten verwendet werden.

Gegenstand der Erfindung ist daher ein Verfahren zur Abtrennung von Isocyansäure aus einem gasförmigen Gemisch aus Isocyansäure und Ammoniak, das dadurch gekennzeichnet ist, daß in ein gasförmiges Gemisch einer Temperatur von 250 bis 600 °C aus Isocyansäure und Ammoniak ein tertiäres Amin oder ein Ether eingebracht und das entstandene gasförmige Reaktionsgemisch in ein unter den Reaktionsbedingungen inertes Verdünnungsmittel eingeleitet und abgekühlt wird, wobei ein Addukt aus Isocyansäure und tertiärem Amin oder Ether kondensiert.

Das zur Durchführung des erfindungsgemäßen Verfahrens nötige Gemisch aus Isocyansäure und Ammoniak wird z.B. durch Zersetzung von Harnstoff hergestellt und gegebenenfalls mit einem Trägergas wie Stickstoff, Argon, Ammoniak verdünnt. Das Gemisch wird anschließend mit einem tertiären Amin oder mit einem Ether versetzt.

Geeignete tertiäre Amine sind beispielsweise tertiäre cyclische Amine wie N-Alkylpyrrolidin, N-Alkylpyrrol, N-Alkylpiperidin, Pyridin, N-Alkyl-Morpholin oder Amine der allgemeinen Formel NR₁R₂R₃, in denen die Reste R₁, R₂ und R₃ unabhängig voneinander geradkettige oder verzweigte Alkyl-, Aryl-, Alkylaryl- oder Arylalkylgruppen bedeuten.
Geeignete Ether sind beispielsweise cyclische Ether, wie Furan, Pyran, Tetrahydrofuran, Dioxan oder Ether der allgemeinen Formel R₁-O-(R₄-O)ₙ-R₂, in der die Reste R₁ und R₂ die oben angegebene Bedeutung haben und R₄ eine geradkettige oder verzweigte Alkylen-, Arylen-, Alkylarylen- oder Arylalkylengruppe und n eine ganze Zahl von 0 bis 5 bedeutet.
Geradkettige oder verzweigte Alkylgruppen sind beispielsweise Alkylgruppen mit 1 bis 10 C-Atomen, wie Methyl-, Ethyl-, Propyl-, Butylgruppen und ihre Isomeren, wie iso-Propyl-, iso-Butyl-, tert. Butylgruppen. Aryl-, Alkylaryl- oder Arylalkylgruppen sind gegebenenfalls ein oder mehrfach durch geradkettige oder verzweigte Alkylgruppen mit 1 bis 5 C-Atomen substituierte Phenylgruppen, die mit dem Stickstoffatom oder dem Ethersauerstoffatom entweder über ein aromatisches oder über ein aliphatisches Kohlenstoffatom verbunden sein können. Beispiele solcher Gruppen sind etwa Phenyl-, Tolyl-, Dimethylphenyl-, Trimethylphenyl-, Ethylphenyl-, Isopropylphenyl-, Benzyl-, Methylbenzyl-, Ethylenphenylgruppen. Alkylengruppen sind beispielsweise Alkylengruppen mit 1 -10 C-Atomen, wie Methylen-, Ethylen-, Propylen-, Butylengruppen und ihre Isomeren, wie isoPropylen-, iso- Butylen-, tert.-Butylengruppen. Arylen-, Alkylarylen-, oder Arylalkylengruppen sind gegebenenfalls ein oder mehrfach durch geradkettige oder verzweigte Alkyl- oder Alkylengruppen mit 1-5 C-Atomen substituierte Phenyl- oder Phenylengruppen, die mit dem Ethersauerstoffatom entweder über ein aromatisches oder über ein aliphatisches Kohlenstoffatom verbunden sein können. Beispiele solcher Gruppen sind Phenylen-, Methylphenylen-, Benzylen-, Dimethylphenylen-, Trimethylphenylen-, Ethylphenylen-, iso-Propylphenylen-, Methylbenzylen-, Ethylenphenylgruppen.
Bevorzugte tertiäre Amine sind tertiäre Amine der allgemeinen Formel NR₁R₂R₃, in denen R₁, R₂ und R₃ gleich sind und eine Alkylgruppe bedeuten. Besonders bevorzugt sind dabei Alkylgruppen mit 1 bis 5 C-Atomen, beispielsweise Trimethylamin, Triethylamin, Tripropylamin, Tributylamin und Triisopentylamin. Ganz besonders bevorzugt sind Trimethylamin, Triethylamin und Triisopentylamin.
Bevorzugte Ether sind Ether der allgemeinen Formel R₁-O-R₂, in der R₁ und R₂ gleich sind und Alkylgruppen bedeuten oder Ether der Formel R₁-O-R₄-O-R₂, in der R₁ und R₂ gleich sind und Alkylgruppen und R₄ eine Alkylengruppe bedeutet.

Das tertiäre Amin oder der Ether werden gegebenenfalls durch Erhitzen gasförmig und gegebenenfalls mit Hilfe eines Trägergases oder flüssig durch Einsprühen oder Eintropfen in das heiße Gasgemisch aus Isocyansäure und Ammoniak eingebracht, wobei das tertiäre Amin oder der Ether in diesem Fall erst im Gasgemisch durch dessen hohe Temperatur in den gasförmigen Zustand übergeführt wird.
Das tertiäre Amin oder der Ether kann dabei etwa äquimolar zur Isocyansäure zugegeben werden, oder es wird ein Überschuß des tertiären Amins oder des Ethers eingesetzt.
Bevorzugt werden pro Mol Isocyansäure 1 bis 7 Mol, besonders bevorzugt 1,5 bis 4 Mol Amin oder Ether eingesetzt.
Die Temperatur, bei der das tertiäre Amin oder der Ether dem gasförmigen Gemisch aus Isocyansäure und Ammoniak zugegeben wird, beträgt etwa 250 bis 600°C, bevorzugt 300 bis 450°C, besonders bevorzugt 320 bis 380°C.

Durch die Zugabe des tertiären Amins oder des Ethers in das Gasgemisch aus Isocyansäure und Ether entsteht ein heißes, gasförmiges Reaktionsgemisch. Die Kontaktzeit der gasförmigen Reaktanten in dem Reaktionsgemisch ist dabei von der Größe der Apparatur und der Strömungsgeschwindigkeit der Gase abhängig, wobei im allgemeinen eine Kontaktzeit von einigen Sekunden ausreichend ist.

Das entstandene heiße, gasförmige Reaktionsgemisch wird anschließend in ein flüssiges, inertes Verdünnungsmittel, das vorgelegt wird, eingeleitet.

Als inerte Verdünnungsmittel kommen beispielsweise aliphatische Kohlenwasserstoffe, wie Pentan, Hexan, Heptan, aromatische Kohlenwasserstoffe, wie Benzol, Toluol, Xylol, halogenierte aliphatische Kohlenwasserstoffe, wie Methylenchlorid, Chloroform, Tetrachlorkohlenstoff, Ethylchlorid, Ethylenchlorid, halogenierte aromatische Kohlenwasserstoffe, wie Chlorbenzol, Trichlorbenzol, Carbonsäureamide, wie Dimethylformamid, N-Methylpyrrolidon oder Ether, wie Diethylether, Diisopropylether, Dibutylether, Ethylmethylether, Dioxan, Diethoxyethan, Tetrahydrofuran oder Mischungen von oben angeführten Verdünnungsmitteln in Frage. Bevorzugt sind aromatische Kohlenwasserstoffe, halogenierte aliphatische Kohlenwasserstoffe und Carbonsäureamide oder Ether, besonders bevorzugt sind Toluol, Chloroform oder N-Methylpyrrolidon oder jener Ether, mit dem die Bildung des Adduktes aus Isocyansäure und Ether erfolgt ist. Dabei dient der Ether sowohl als Reaktant als auch als inertes Verdünnungsmittel.

Beim Einleiten des heißen gasförmigen Reaktionsgemisches in das flüssige inerte Verdünnungsmittel wird das heiße gasförmige Reaktionsgemisch abgekült oder das heiße, gasförmige Reaktionsgemisch wird bereits vor dem Einleiten in das flüssige, inerte Verdünnungsmittel, etwa mit Hilfe von Wärmeaustauschern, vorgekühlt. Das gasförmige Reaktionsgemisch wird beispielsweise durch Einleiten, über Füllkörperkolonnen, Wäscher usw. in das flüssige inerte Verdünnungsmittel eingebracht. Dabei kann das flüssige inerte Verdünnungsmittel etwa mit Hilfe von Wärmeaustauschern gekühlt vorgelegt werden.

In dem vorgelegten Verdünnungsmittel kondensiert das Addukt aus Isocyansäure und tertiärem Amin bzw. aus Isocyanäsure und Ether, während das Ammoniak gasförmig entweicht. Zur vollständigen Abtrennung des Ammoniaks kann ein inertes Trägergas, wie Stickstoff, Argon durch die gebildete Lösung oder Suspension durchgeblasen werden.

In einer bevorzugten Ausführungsform wird in das Gemisch aus Isocyansäure und Ammoniak bei Temperaturen von 320 bis 380°C gasförmiges Trimethylamin, Triethylamin, Triisopentylamin oder Dioxan, Diisopropylether, Diphenylether, Diethoxyethan eingeblasen, worauf der heiße Gasstrom in einem Wäscher mit gekühltem Toluol, Chloroform, N-Methylpyrrolidon oder Dioxan, Diisopropylether, Diphenylether, Diethoxyethan durch das Stickstoff durchgeleitet wird, in Kontakt gebracht wird. Dabei kondensiert das Addukt und das gasförmige und das eventuell gelöste Ammoniak wird mit Hilfe des Stickstoffstromes abgetrennt.

Das Verfahren kann kontinuierlich oder diskontinuierlich durchgeführt werden, wird aber bevorzugt kontinuierlich durchgeführt.

Auf die oben beschriebene Art und Weise wird je nach der Natur des verwendeten Verdünnungsmittels bzw. je nach der Natur des Adduktes eine Lösung oder eine Suspension eines Adduktes von Isocyansäure mit einem tertiärem Amin oder von Isocyansäure mit Ether erhalten, die zur Weiterreaktion mit verschiedensten Substraten verwendet werden können. Es kann möglich sein, daß in einer Suspension das Addukt gelöst vorliegt, und ein Feststoff, der nicht Addukt ist, ausgefallen ist. In solchen Fällen wird die Suspension vorteilhafterweise vor der Weiterverwendung filtriert und vom gebildeten Nebenprodukt abgetrennt. Falls ein Ether-Isocyansäure-Addukt hergestellt wurde, kann gegebenenfalls eine Reinigung durch Destillation erfolgen.

Durch Umsetzung des Adduktes in einem unter den Reaktionsbedingungen inerten Verdünnungsmittel bei Temperaturen von -20 °C bis zum Siedepunkt des verwendeten Verdünnungsmittels mit einem primären oder sekundären Amin wird ein unsymmetrisch substituierter Harnstoff, mit einem Alkohol ein Carbamat, mit einem Thiol ein Thiocarbamt und mit einer Verbindung, die eine oder zwei nicht kumulierte, olefinische Doppelbindungen enthält, ein substituiertes Isocyanat erhalten.

### Beispiel 1

100 g Harnstoff wurden pro Stunde kontinuierlich in einen Zersetzer eingetragen. Die Pyrolysegase wurden in einem beheizbaren Rohr bei 320°C mit 255 g Triethylamin pro Stunde, das gasförmig eingebracht wurde, zur Reaktion gebracht. Insgesamt wurde 213 g Harnstoff (3,5 Mol) und 544 g Triethylamin (5,4 Mol) eingetragen. Die Reaktionsgase wurden in einem Wäscher, der mit Chloroform, das auf -10 °C gekühlt war, betrieben wurde, rasch auf Raumtemperatur abgekühlt. Die organische Phase wurde filtriert.
Dabei wurden 66 % der Theorie eines Adduktes aus Isocyansäure und **Triethylamin**, gelöst in Chloroform, erhalten.
IR: 2160 cm⁻¹ (N=C=O), (scharfe Bande)
Der Gehalt der Lösung an Addukt wurde durch saure Hydrolyse und Bestimmung des entweichenden CO₂ mit Hilfe von Barytlauge bestimmt.

### Bespiel 2

60 g Harnstoff wurden pro Stunde kontinuierlich in einen Zersetzer eingetragen. Die Pyrolysegase wurden in einem beheizbaren Rohr bei 320°C mit 285 g Triethylamin pro Stunde, das gasförmig eingebracht wurde, zur Reaktion gebracht. Insgesamt wurden 71 g Harnstoff (1,2 Mol) und 388 g Triethylamin (3,3 Mol) eingetragen.
Die Reaktionsgase wurden in einem Wäscher, der mit Toluol betrieben wurde, rasch auf Raumtemperatur abgekühlt.
Dabei wurden 55 % der Theorie eines Adduktes aus Isocyansäure und Triethylamin, suspendiert in Toluol, erhalten. Die Ausbeute wurde, wie in Beispiel 1 beschrieben, bestimmt.
IR: 2160 cm⁻¹ (N=C=O), (scharfe Bande)

### Beispiel 3

Wie im Beispiel 1 beschrieben, aber unter Verwendung von 100 g Harnstoff (1,7 Mol), 200 g Triethylamin (1,98 Mol) und N-Methylpyrrolidon, das auf -10 °C gekühlt war, als Verdünnungsmittel wurden 62 % der Theorie eines Adduktes aus **Isocyansäure** und **Triethylamin** erhalten.
IR: 2160 cm⁻¹ (N=C=O), scharfe Bande

### Beispiel 4

Wie im Beispiel 1 beschrieben, aber unter Verwendung von 80 g Harnstoff (1,3 Mol), 160 g Triethylamin (1,58 Mol) und n-Hexan, das auf -20 °C gekühlt war, als Verdünnungsmittel wurden 52 % der Theorie eines Adduktes aus **Isocyansäure** und **Triethylamin** erhalten.
IR: 2160 cm⁻¹ (N=C=O), scharfe Bande

### Beispiel 5

Wie im Beispiel 1 beschrieben, aber unter Verwendung von 252,2 g Harnstoff (4,2 Mol) und 796 g Triisopentylamin (3,5 Mol) wurden nach dem Filtrieren der entstandenen Suspension 50 % der Theorie eines Adduktes aus **Isocyansäure** und **Triisopentylamin** erhalten.
IR: 2160 cm⁻¹ (N=C=O), scharfe Bande

### Beispiel 6

Wie im Beispiel 2 beschrieben, aber unter Verwendung von 305 g N,N-Dimethylcyclohexylamin (2,4 Mol) und Chloroform einer Temperatur von -10 °C als Verdünnungsmittel, wurden nach dem Filtrieren der entstandenen Suspension 62 % der Theorie eines Adduktes aus **Isocyansäure** und **N,N-Dimethylcyclohexylamin**, gelöst in Chloroform, erhalten.
IR: 2160 cm⁻¹, (N=C=O), scharfe Bande

### Beispiel 7

Wie im Beispiel 1 beschrieben aber unter Verwendung von 71 g Harnstoff (1,2 Mol) und 238 g N-Methylpiperidin (2,4 Mol) und Chloroform, das auf -10 °C gekühlt war, als Verdünnungsmittel, wurden 51 % der Theorie eines Adduktes aus **Isocyansäure** und **N-Methylpiperidin**, gelöst in Chloroform, erhalten.
IR: 2160 cm⁻¹ (N=C=O) scharfe Bande

### Beispiel 8

90 g Harnstoff wurden pro Stunde kontinuierlich in einen Zersetzer eingetragen. Die Pyrolysegase wurden in einem beheizbaren Rohr bei 360 °C mit 372 g N,N-Dimethylanilin pro Stunde, das gasförmig mit Hilfe eines Stickstoffstromes eingebracht wurde, zur Reaktion gebracht. Insgesamt wurden 135 g Harnstoff (2,3 Mol) und 557 g N,N-Dimethylanilin (4,6 Mol) eingetragen. Die Reaktionsgase wurden in einem Wäscher, der mit Chloroform betrieben wurde und auf -15° C gekühlt war, rasch abgekühlt. Die entstandene Suspension wurde filtriert. Dabei wurden 58 % der Theorie eines Adduktes aus **Isocyansäure** und **N,N-Dimethylanilin**, gelöst in Chloroform, erhalten. Die Ausbeute wurde wie in Beispiel 1 beschrieben bestimmt.
IR: 2160 cm⁻¹ (N=C=O), scharfe Bande

### Beispiel 9

80 g Harnstoff wurden pro Stunde kontinuierlich in einem Zersetzer eingetragen. Die Pyrolysegase wurden in einem beheizbaren Rohr bei 340 °C mit 217 g N-Benzyldimethylamin pro Stunde, das mit Hilfe von Stickstoff gasförmig eingebracht wurde, zur Reaktion gebracht.
Insgesamt wurden 96,1 g Harnstoff (1,6 Mol) und 242 g N-Benzyldimethylamin (2,0 Mol) eingetragen.
Die Reaktionsgase wurden in einem Wäscher, der mit Chloroform, das auf -15 °C gekühlt war, betrieben wurde, abgekühlt. Die entstandene Suspension wurde filtriert.
Dabei wurden 61 % der Theorie eines Adduktes aus **Isocyansäure** und **N-Benzyldimethylamin**, gelöst in Chloroform, erhalten. Die Ausbeute wurde wie im Beispiel 1 beschrieben bestimmt.
IR: 2160 cm⁻¹ (N=C=O), scharfe Bande

### Beispiel 10

80 g Harnstoff wurden pro Stunde kontinuierlich in einen Zersetzer eingetragen. Die Pyrolysegase wurden bei 360 °C in einem beheizten Rohr mit 120 g gasförmigen Dioxan pro Stunde zur Reaktion gebracht. Insgesamt wurden 400 g Harnstoff (6,66 Mol) und 600 g Dioxan (6,81 Mol) eingesetzt. Die Reaktionsgase wurden in einem mit Dioxan betriebenen Wäscher bei 10 °C abgekühlt. Das entstandene, gelöste Addukt wurde von einem ausgefallenen Feststoff abfiltriert.
Dabei wurden 1700 g einer Lösung erhalten, die 24%ig an **Isocyansäure-Dioxan-Addukt**, das entspricht einer Ausbeute von 47 % der Theorie, war. Die Ausbeute wurde, wie in Beispiel 1 beschrieben, bestimmt.
IR: 2160 cm⁻¹ (N=C=O), scharfe Bande

### Beispiel 11

80 g Harnstoff wurden pro Stunde kontinuierlich in einen Zersetzer eingetragen. Die Pyrolysegase wurden bei 350 °C in einem beheizten Rohr mit 306 g gasförmigem Diisopropylether pro Stunde zur Reaktion gebracht. Insgesamt wurden 160 g Harnstoff (2,66 Mol) und 612 g Diisopropylether (5,99 Mol) eingesetzt. Die Reaktionsgase wurden in einem mit Diisopropylether betriebenen Wäscher bei 10 °C abgekühlt. Das entstandene, gelöste Addukt wurde von einem ausgefallenen Feststoff abfiltriert.
Dabei wurden 1700 g einer Lösung erhalten, die 15%ig an **Isocyansäure-Diisopropylether-Addukt**, das entspricht einer Ausbeute von 70 % der Theorie, war. Die Ausbeute wurde, wie in Beispiel 1 beschrieben, bestimmt.
IR: 2160 cm⁻¹ (N=C=O), scharfe Bande

### Beispiel 12

40 g Harnstoff wurden pro Stunde kontinuierlich in einen Zersetzer eingetragen. Die Pyrolysegase wurden bei 370 °C in einem beheizten Rohr mit 113 g gasförmigen Diphenylether pro Stunde zur Reaktion gebracht. Insgesamt wurden 160 g Harnstoff (2,66 Mol) und 452 g Diphenylether (3,51 Mol) eingesetzt. Die Reaktionsgase wurden in einem mit Chloroform betriebenen Wäscher bei 10 °C abgekült. Die entstandene Suspension wurde von einem ausgefallenen Feststoff abfiltriert.
Dabei wurden 2000 g einer Lösung erhalten, die 17%ig an **Isocyansäure-Diphenylether-Addukt**, das entspricht einer Ausbeute von 60 % der Theorie, war. Die Ausbeute wurde, wie in Beispiel 1 beschrieben, bestimmt.
IR: 2160 cm⁻¹ (N=C=O), scharfe Bande

### Beispiel 13

120 g Harnstoff wurden pro Stunde kontinuierlich in einen Zersetzer eingebracht. Die Pyrolysegase wurden bei 380°C in einem beheizten Rohr mit 350 g gasförmigem Diethoxyethan pro Stunde zur Reaktion gebracht. Insgesamt wurden 120 g (2 Mol) Harnstoff und 350 g (3 Mol) Diethoxyethan eingesetzt. Die Reaktionsgase wurden in einem mit Diethoxyethan betriebenen Wäscher bei 10 °C rasch abgekült.
Die Lösung des Isocyansäure-Diethoxyethan-Addukts in Diethoxyethan wurde anschließend destilliert.
Bei der Destillation wurden zwischen 70 - 110°C unter Normaldruck 270 ml einer Fraktion erhalten, die 74%ig an **Isocyansäure-Diethoxyethan-Addukt** waren, das entspricht 62 % der Theorie. Die Bestimmung der Ausbeute erfolgte wie im Beispiel 1 beschrieben.
IR: 2160 cm⁻¹ (N=C=O), scharfe Bande

### Beispiel 14

In 100 ml einer Lösung von 10 g Triethylammoniumisocyanat (0,069 Mol) in Chloroform, hergestellt nach Beispiel 1, wurden bei Raumtemperatur 14,1 g Dodecylamin (0,076 Mol) gelöst in 20 ml Chloroform unter Rühren zugetropft. Nach beendeter Zugabe wurde 24 Stunden bei Raumtemperatur nachgerührt und 1 Stunde auf Rückfluß erhitzt. Das Lösungsmittel wurde abgedampft und der Rückstand aus Chloroform umkristallisiert. Dabei wurden 9,45 g, das sind 60 % der Theorie, **Dodecylharnstoff** erhalten.

| | | | | |
|---|---|---|---|---|
| C-H-N-Analyse: | theoret.: | C 68,4 %, | H 12,3 %, | N 12,3 % |
| | gef.: | C 68,2 %, | H 12,3 %, | N 12,3 % |

### Beispiel 15

In 100 ml einer Lösung von 10,5 g (0,073 Mol) Triethylammomiumisocyanat in Chloroform, hergestellt nach Beispiel 1, wurden 6,7 g (0,146 Mol) Ethanol bei Raumtemperatur unter Rühren getropft, worauf 24 Stunden bei Raumtemperatur nachgerührt und anschließend 1 Stunde auf Rückfluß erhitzt wurde.
Das Lösungsmittel wurde abgedampft und der Rückstand aus Ethanol umkristallisiert, wobei 4,6 g, das sind 71 % der Theorie **Ethylcarbamat** mit einem Schmelzpunkt von 46 - 50°C erhalten wurden.

### Beispiel 16

In 120 ml einer Lösung von 28 g (0,2 Mol) Triethylammoniumisocyanat in Chloroform, hergestellt nach Beispiel 1, wurden 18,5 g (0,3 Mol) Ethylmercaptan, gelöst in 30 ml Chloroform, bei 0°C unter Rühren getropft, worauf noch 1 Stunde bei dieser Temperatur und anschließend 15 Stunden bei Raumtemperatur nachgerührt wurde. Hierauf wurde die Reaktionsmischung 2 Stunden unter Rückfluß erhitzt. Das Lösungsmitel wurde abdestilliert. Der verbleibende ölige Rückstand kristallisierte beim Abkühlen und wurde aus Wasser umkristallisiert.
Dabei wurden 16 g (0.15 Mol), das sind 75 % der Theorie **Thiocarbamidsäure-S-ethylester** mit einem Schmelzpunkt von 99 - 102 °C erhalten

### Beispiel 17

In 100 ml einer Suspension von 11,3 g (0,078 Mol) Triethylammoniumisocyanat in Toluol wurden unter Rühren 3 g (0,025 Mol) alpha-Methylstyrol getropft, worauf 3 Stunden bei Raumtemperatur nachgerührt und anschließend circa 4 Stunden auf Rückfluß erhitzt wurden.
Dabei wurde eine Lösung von alpha,alpha-Dimethylbenzylisocyanat in Toluol erhalten.
Nach Destillation bei 40 bis 45 °C, 1 Torr wurde alpha,alpha-**Dimethylbenzylisocyanat** mit einem n_{D}²⁵ von 1,5048 in einer Ausbeute von 55 % der Theorie erhalten.

## Patentansprüche

1. Verfahren zur Abtrennung von Isocyansäure aus einem gasförmigen Gemisch aus Isocyansäure und Ammoniak, dadurch gekennzeichnet, daß in ein gasförmiges Gemisch einer Temperatur von 250 bis 600 °C aus Isocyansäure und Ammoniak ein tertiäres Amin oder ein Ether eingebracht und das entstandene gasförmige Reaktionsgemisch in ein unter den Reaktionsbedingungen inertes Verdünnungsmittel eingeleitet und abgekült wird, wobei ein Addukt aus Isocyansäure und tertiärem Amin oder Ether kondensiert.

2. Verfahren nach Anspruch 1, dadurch gekennzeichnet, daß ein tertiäres cyclisches Amin oder ein Amin der allgemeinen Formel NR₁R₂R₃, in der die Reste R₁, R₂ und R₃ unabhängig voneinander geradkettige oder verzweigte Alkyl-, Aryl-, Alkylaryl- oder Arylalkylgruppen bedeuten, eingesetzt wird.

3. Verfahren nach Anspruch 2, dadurch gekennzeichnet, daß ein Trialkylamin eingesetzt wird.

4. Verfahren nach Anspruch 1, dadurch gekennzeichnet, daß ein cyclischer Ether oder ein Ether der allgemeinen Formel R₁-0-(R₄-0)ₙ-R₂, in der die Reste R₁ und R₂ unabhängig voneinander eine geradkettige oder verzweigte Alkyl-, Aryl-, Alkylaryl- oder Arylalkylgruppe, R₄ eine geradkettige oder verzweigte Alkylen-, Arylen-, Alkylarylen- oder Arylalkylengruppe und n eine ganze Zahl von 0 bis 5 bedeutet, eingesetzt wird.

5. Verfahren nach Anspruch 4, dadurch gekennzeichnet, daß als Ether ein Ether der Formel R₁-O-R₂, in der R₁ und R₂ gleich sind und Alkylgruppen bedeuten oder ein Ether der Formel R₁-O-R₄-O-R₂, in der R₁ und R₂ die oben angegebenen Bedeutung haben und R₄ eine Alkylengruppe bedeutet, eingesetzt wird.

6. Verfahren nach einem der Ansprüche 1 bis 4, dadurch gekennzeichnet, daß pro Mol Isocyansäure 1 bis 7 Mol tertiäres Amin oder Ether eingebracht werden.

7. Verfahren nach einem der Ansprüche 1 bis 5, dadurch gekennzeichnet, daß die Zugabe des tertiären Amins oder des Ethers bei einer Temperatur von 320 bis 380°C erfolgt.

8. Verfahren nach einem der Ansprüche 1 bis 4, dadurch gekennzeichnet, daß als Verdünnungsmittel ein gegebenenfalls halogenierter aliphatischer oder aromatischer Kohlenwasserstoff, ein Ether, ein Carbonsäureamid oder eine Mischung von oben angeführten Verdünnungsmitteln eingesetzt wird.

9. Verfahren nach einem der Ansprüche 4 oder 5, dadurch gekennzeichnet, daß als Verdünnungsmittel der Ether eingesetzt wird, der auch das Addukt aus Isocyansäure und Ether bildet.

10. Verfahren zur Herstellung von unsymmetrisch substituierten Harnstoffen, von Carbamaten, von Thiocarbamaten oder von substituierten Isocyanaten, dadurch gekennzeichnet, daß ein Addukt aus Isocyansäure und einem tertiärem Amin oder einem Ether, hergestellt nach einem der Ansprüche 1 bis 9 in einem inerten Verdünnungsmittel bei Temperaturen von -20 °C bis zum Siedepunkt des Verdünnungsmittel mit einem primären oder sekundären Amin, einem Alkohol, einem Thiol oder einer Verbindung, die eine oder zwei nicht kumulierte, olefinische Doppelbindungen enthält, umgesetzt wird.

## Claims

1. Process for the removal of isocyanic acid from a gaseous mixture of isocyanic acid and ammonia, characterized in that a tertiary amine or an ether is introduced into a gaseous mixture of isocyanic acid and ammonia at a temperature of 250 to 600°C and the resulting gaseous reaction mixture is introduced into a diluent which is inert under the reaction conditions and cooled, an adduct of isocyanic acid and tertiary amine or ether condensing.

2. Process according to Claim 1, characterized in that a tertiary cyclic amine or an amine of the general formula NR₁R₂R₃ in which the radicals R₁, R₂ and R₃ independently of one another denote straight-chain or branched alkyl, aryl, alkylaryl or arylalkyl groups, is employed.

3. Process according to Claim 2, characterized in that a trialkylamine is employed.

4. Process according to Claim 1, characterized in that a cyclic ether or an ether of the general formula R₁-O-(R₄-O)ₙ-R₂ in which the radicals R₁ and R₂ independently of one another denote a straight-chain or branched alkyl, aryl, alkylaryl or arylalkyl group, R₄ denotes a straight-chain or branched alkylene, arylene, alkylarylene or arylalkylene group and n denotes an integer from 0 to 5, is employed.

5. Process according to Claim 4, characterized in that an ether of the formula R₁-O-R₂ in which R₁ and R₂ are identical and denote alkyl groups, or an ether of the formula R₁-O-R₄-O-R₂ in which R₁ and R₂ have the above-mentioned meaning and R₄ denotes an alkylene group, is employed as the ether.

6. Process according to one of Claims 1 to 4, characterized in that 1 to 7 moles of tertiary amine or ether are introduced per mole of isocyanic acid.

7. Process according to one of Claims 1 to 5, characterized in that the addition of the tertiary amine or the ether is carried out at a temperature of 320 to 380°C.

8. Process according to one of Claims 1 to 4, characterized in that an optionally halogenated aliphatic or aromatic hydrocarbon, an ether, a carboxamide or a mixture of the abovementioned diluents is employed as the diluent.

9. Process according to one of Claims 4 or 5, characterized in that the ether which also forms the adduct of isocyanic acid and ether is employed as the diluent.

10. Process for the preparation of asymmetrically substituted ureas, carbamates, thiocarbamates or substituted isocyanates, characterized in that an adduct of isocyanic acid and a tertiary amine or an ether, prepared according to one of Claims 1 to 9 in an inert diluent at temperatures of -20°C up to the boiling point of the diluent, is reacted with a primary or secondary amine, an alcohol, a thiol or a compound which contains one or two non-cumulated olefinic double bonds.

## Revendications

1. Procédé de séparation de l'acide isocyanique dans un mélange gazeux d'acide isocyanique et d'ammoniac, caractérisé en ce qu'on introduit une amine tertiaire ou un éther dans un mélange gazeux d'acide isocyanique et d'ammoniac à une température de 250 à 600°C, on introduit le mélange de réaction gazeux formé dans un diluant inerte dans les conditions de réaction et on le refroidit, et un produit d'addition d'acide isocyanique et d'amine tertiaire ou d'éther est condensé.

2. Procédé selon la revendication 1, caractérisé en ce qu'on utilise une amine tertiaire cyclique ou une amine répondant à la formule générale NR₁R₂R₃ dans laquelle les restes R₁, R₂ et R₃ signifient, indépendamment les uns des autres, des groupes alkyles, aryles, alkylaryles ou arylalkyles à chaîne droite ou ramifiée.

3. Procédé selon la revendication 2, caractérisé en ce qu'on utilise une trialkylamine.

4. Procédé selon la revendication 1, caractérisé en ce qu'on utilise un éther cyclique ou un éther répondant à la formule générale R₁-O-(R₄-O)ₙ-R₂ dans laquelle les restes R₁ et R₂ signifient, indépendamment l'un de l'autre, un groupe alkyle, aryle, alkylaryle ou arylalkyle à chaîne droite ou ramifiée, R₄ signifie un groupe alkylène, arylène, alkylarylène ou arylalkylène à chaîne droite ou ramifiée, et n signifie un nombre entier de 0 à 5.

5. Procédé selon la revendication 4, caractérisé en ce qu'on utilise, comme éther, un éther répondant à la formule R₁-O-R₂ dans laquelle R₁ et R₂ sont identiques et signifient des groupes alkyles, ou un éther répondant à la formule R₁-O-R₄-O-R₂ dans laquelle R₁ et R₂ ont la signification indiquée ci-dessus, et R₄ signifie un groupe alkylène.

6. Procédé selon l'une des revendications 1 à 4, caractérisé en ce qu'on introduit 1 à 7 moles d'amine tertiaire ou d'éther par mole d'acide isocyanique.

7. Procédé selon l'une des revendications 1 à 5, caractérisé en ce qu'on effectue l'addition de l'amine tertiaire ou de l'éther à une temperature de 320 à 380°C.

8. Procédé selon l'une des revendications 1 à 4, caractérisé en ce qu'on utilise, comme diluant, un hydrocarbure aliphatique ou aromatique éventuellement halogéné, un éther, un amide carboxylique ou un mélange des diluants indiqués ci-dessus.

9. Procédé selon l'une des revendications 4 ou 5, caractérisé en ce qu'on utilise, comme diluant, l'éther qui forme aussi le produit d'addition d'acide isocyanique et d'éther.

10. Procédé de préparation d'urées substituées de façon asymétrique, de carbamates, de thiocarbamates ou d'isocyanates substitués, caractérisé en ce qu'on fait réagir un produit d'addition d'acide isocyanique et d'une amine tertiaire ou d'un éther, préparé selon l'une des revendications 1 à 9, dans un diluant inerte, à des températures comprises entre -20°C et le point d'ébullition du diluant, avec une amine primaire ou secondaire, un alcool, un thiol ou un composé qui contient une ou deux liaisons doubles oléfiniques non cumulées.
